# EUROPEAN PATENT SPECIFICATION

(11) **EP 3 865 093 B1**
(45) Date of publication and mention of the grant of the patent: **27.09.2023**
(21) Application number: 19870367.0
(22) Date of filing: 07.10.2019
(51) Int. Cl.: A61F 2/24, A61F 2/76

(54) **TEMPLATE**
SCHABLONE
MODÈLE

(30) Priority: 09.10.2018 JP 2018190673
(43) Date of publication of application: 18.08.2021
(73) Proprietor: Ozaki, Shigeyuki, Tokyo 152-0032 (JP)
(72) Inventor: Ozaki, Shigeyuki, Tokyo 152-0032 (JP)
(74) Representative: Bandpay & Greuter
(86) International application number: PCT/JP2019/039524
(87) International publication number: WO 2020/075683

(56) References cited:
- EP-A1- 3 120 809
- JP-A- 2008 073 538
- JP-A- 2015 181 521
- JP-U- H02 142 568
- US-A1- 2010 018 447
- US-A1- 2017 340 439
- US-A1- 2017 340 439

## Description

### Technical Field

The present invention relates to a template.

### Background Art

As a template for forming an aortic valve from a pericardium, there is, for example, a template disclosed in Patent Document 1. This template includes a valve cusp base forming part, which is a base of a valve cusp and holes indicating a portion to be a suture allowance, and a wing forming part, which is outside the valve cusp base forming part and corresponds to a hole or holes for forming a wing part to be fixed to a blood vessel. In addition, this template has a plurality of scale marks that indicate the points where suturing is performed during a procedure on the outside of the valve cusp base forming part. When forming an aortic valve from the pericardium, this template is used to draw lines along the valve cusp base forming part with a surgical skin marker and to draw marks at the hole positions of the wing forming part. Thereafter, the pericardium is cut with reference to the drawn marks at the hole positions of the wing forming part and the lines, by which a valve cusp material to be a valve cusp is formed.

### Citation List

### Patent Document

Patent Document 1: Japanese Patent Application Laid-Open No. 2015-181521 .The document US 2017/340439 A1 relates to equipment for preparing valve leaflet from membrane.

### Summary of the Invention

### Problems to be solved by the Invention

In the case of the template disclosed in Patent Document 1, marks are drawn with reference to the scale marks provided on the outside of the valve cusp base forming part, by which the drawn marks serve as marks for positions where the valve cusp material is sutured to the aorta. Since the position of the edge of the valve cusp base forming part corresponds to a part to be a suture allowance, however, drawing the marks at the actual suturing positions requires determining the suture allowance from the scale marks present along the edge of the valve cusp base forming part by eye before drawing the marks at positions separated in the radial direction by the suture allowance determined by eye. In this case, due to the lack of skill of a user or the misunderstanding of the meaning of the scale marks, it was likely that the marks would not be drawn at appropriate positions. Suturing according to inappropriately positioned marks may affect the function of the valve cusp formed by the suture.

An object of the present invention is to assist in drawing marks indicating suture positions at appropriate positions in a pericardium.

### Means for solving the Problems

The present invention provides a template according to claim 1 for forming an aortic valve from a pericardium including: a plurality of first holes for drawing lines indicating positions at which a membrane is to be cut, each first hole having a linear straight line part and an arc part; and a plurality of second holes that are provided at a predetermined distance from the plurality of first holes and are for use in drawing marks at suture positions where the cut membrane is to be sutured.

The plurality of second holes includes a hole for drawing a mark indicating a start position of suturing a valve cusp material formed from the pericardium.

The plurality of second holes includes holes for use in drawing a mark indicating a suture position in suturing the valve cusp material to an adjacent valve cusp.

The plurality of second holes includes a plurality of holes, between the hole for drawing a mark indicating a start position and holes for use in drawing a mark indicating a suture position in suturing the valve cusp material to an adjacent valve cusp, the positions of the plurality of holes corresponding to the suture positions in suturing the valve cusp material from the start position of suturing the valve cusp material toward a commissure part.

The plurality of second holes includes holes for use in drawing a mark indicating the suture position in forming the commissure part.

The hole for drawing a mark indicating a start position differs in at least shape or size from the hole for drawing a mark at a position other than the start position.

In an example of the template according to claim 2, the holes for use in drawing the mark indicating a suture position in suturing the valve cusp material to an adjacent valve cusp may differ in at least shape or size from the plurality of holes corresponding to the suture positions in suturing the valve cusp material from the start position of suturing the valve cusp material toward a commissure part and from the holes for use in drawing a mark indicating the suture position in forming the commissure part.

In an example, the template may be configured so that the template has the second hole between the second hole for drawing the mark at the second position and the first hole.

In an example of the template according to claim 3, the present invention may be configured so that the portion other than the first holes and the second holes is transparent.

### Advantageous Effect of the Invention

The present invention enables assisting in drawing marks indicating suture positions at appropriate positions in a pericardium.

### Brief Description of the Drawings

Fig. 1 is a diagram illustrating an example of a template 1 according to the present invention.
Fig. 2 is an enlarged view of a forming part 2.
Fig. 3 is a diagram illustrating an example of lines and marks drawn on the pericardium by using the template 1 and a skin marker 5.
Fig. 4 is a diagram illustrating an example of a valve cusp material 6 obtained by cutting the pericardium
Fig. 5 is a diagram for describing suturing of the valve cusp material 6 to an aorta 7.
Fig. 6 is a diagram for describing suturing of the valve cusp material 6 to the aorta 7.
Fig. 7 is a diagram illustrating an example of the forming part 2 according to a modification.

### Mode for carrying out the Invention

### [Embodiments]

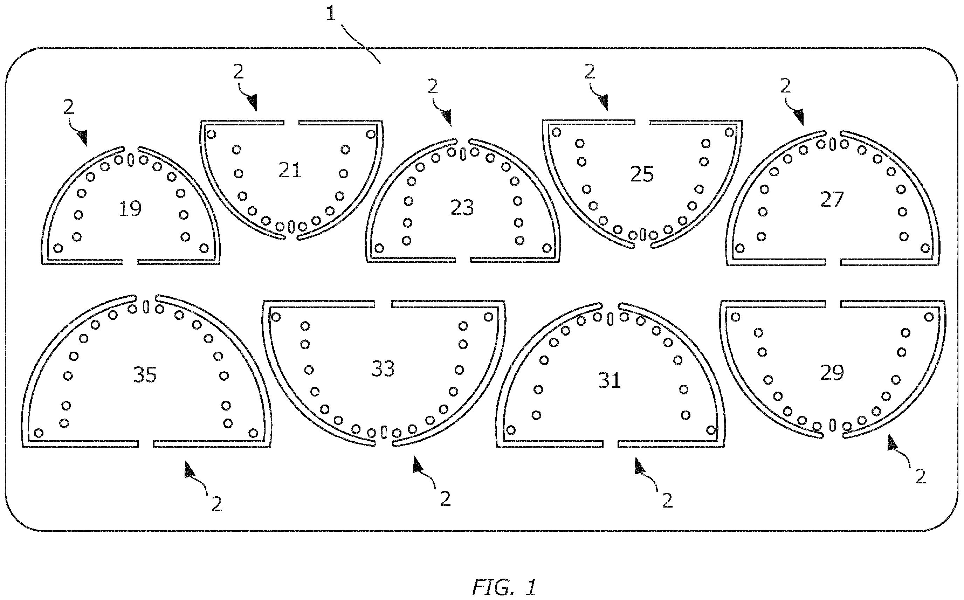

Fig. 1 is a diagram illustrating an example of a template 1 according to the present invention. The template 1 is for use in aortic valvuloplasty and is used to cut the pericardium taken from a patient to form a valve cusp of an aorta of the patient. The template 1 is transparent, so that the pericardium under the template 1 can be seen when placed on the top of the pericardium taken from the patient. The template 1 includes a plurality of forming parts 2 to accommodate various sizes of valve cusps to be formed from the pericardium.

When forming a valve cusp material that is to be used as a valve cusp from the taken pericardium, for example, the size of the valve cusp is measured using a plurality of valve cusp sizers disclosed in Japanese Patent Application Laid-Open No. 2016-54827. The valve cusp sizer is used to measure a distance from one commissure part where the excised valve cusp has been present to the other commissure part on the inner circumference of the blood vessel in the aorta from which the valve cusp was excised. The valve cusp sizer has an arcuate surface when the cylinder is cut at an angle (for example, 120 degrees) corresponding to the central angle of the commissure part of the aortic valve. Regarding the size of the valve cusp sizer, for example, the diameter of the cylindrical shape before being divided into three equal parts is used as a nominal diameter for specifying the size of the valve cusp, and the nominal diameter is an odd number from 17 to 35. The number printed on each forming part 2 indicates the size of the valve cusp and corresponds to the nominal diameter value of the valve cusp sizer described above. The size of the forming part 2 is not limited to the above-described size, and the template 1 may have a configuration including the forming part 2 for a size other than the above-described size. Moreover, the nominal diameter of the valve cusp sizer and the size of the forming part 2 may be even numbers, and may have both odd and even numbers.

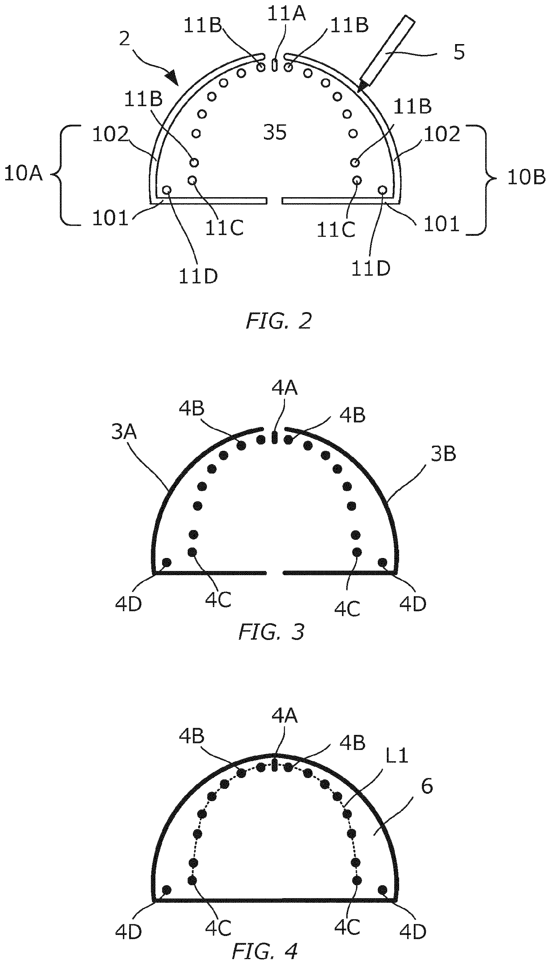

Subsequently, the configuration of each forming part 2 will be described by giving an example of an enlarged forming part 2 illustrated in Fig. 2. Each of the plurality of forming parts 2 has a hole 10A and a hole 10B for drawing lines which indicate the positions at which the pericardium taken from a patient is to be cut. The hole 10A and the hole 10B are merely examples of first holes according to the present invention. In this embodiment, the hole 10A and the hole 10B have a symmetrical relationship. The hole 10A and the hole 10B each have a linear straight line part 101 and an arc part 102 on an arc. The lengths of the straight line part 101 and the arc part 102 are different for each forming part 2.

In addition, each of the plurality of forming parts 2 has a hole 11A, a plurality of holes 11B, holes 11C, and holes 11D for use in drawing marks indicating positions at which valve cusp material cut and formed from the pericardium is sutured to a blood vessel. The hole 11A, the plurality of holes 11B, holes 11C, and holes 11D are examples of the second holes according to the present invention. The hole 11A, the plurality of holes 11B, holes 11C, and holes 11D are provided at a predetermined distance from the hole 10A and the holes 10B.

The hole 11A is a hole for drawing a mark indicating the start position (first position) of suturing the valve cusp material formed from the pericardium, and has a different shape from the hole 11B, the hole 11C, and the hole 11D. Although the hole 11A has an oval shape in this embodiment, the shape is not limited to the oval shape, and any other shape may be applied as long as it is different from the shape of the hole 11B, the hole 11C, and the hole 11D. In addition, the hole 11A may be similar in shape to the hole 11B, the hole 11C, and the hole 11D, and may be larger than the hole 11B, the hole 11C, and the hole 11D.

The number of the plurality of holes 11B between the hole 11A and the hole 11C corresponds to the size of the valve cusp printed on the forming part 2. The positions of the holes 11B correspond to the suture positions in suturing the valve cusp material from the start position of suturing the valve cusp material toward the commissure part.

The hole 11C is for use in drawing a mark indicating the suture position in suturing the valve cusp material to the adjacent valve cusp. Moreover, the hole 11D is for use in drawing a mark indicating the suture position in forming a commissure part.

Subsequently, an example of using the template 1 will be described by giving an example of aortic reconstruction, in which an aortic valve is formed by using a valve cusp material formed from a pericardium taken from a patient's heart. In the aortic reconstruction, the sternum is exposed and then a median sternotomy is performed to take the pericardium from the patient's heart. With the heart exposed, an extracorporeal circulation is performed by a heart-lung machine, the heartbeat is stopped, and thereafter the aortic valve is exposed. On the other hand, the pericardium taken from the heart is expanded by a thread and fixedly immersed in a tissue-fixing solution (for example, a solution containing glutaraldehyde).

Subsequently, what is to be removed from the aorta in the valve cusp of the patient's aortic valve is excised. The size of the excised valve cusp is measured by applying the valve cusp sizer described above to the site where the valve cusp has been excised. In the case where a plurality of valve cusps was excised, the valve cusp sizer is applied to each excised site to measure the size of each excised valve cusp. After the completion of measuring the size of the valve cusp, the pericardium is removed from the tissue-fixing solution. Then, the part of the forming part 2 with the length of the arcuate surface of the valve cusp sizer is placed on the fixed pericardium, where the valve cusp sizer matches the distance between the commissure parts of the excised valve cusp.

Then, as illustrated in Fig. 2, the pen tip of a skin marker 5 is inserted into the hole 10A and the hole 10B of the forming part 2, and the skin marker 5 is moved along the hole 10 and the hole 10B to draw lines on the fixed pericardium. This enables drawing of the part of the pericardium that is cut to form the valve cusp material. The lines drawn along the hole 10A and the hole 10B may be continuous lines, dotted lines, or broken lines as the lines only need to be a guide for cutting.

Furthermore, the pen tip of the skin marker 5 is inserted into the hole 11A, the holes 11B, the holes 11C, and the holes 11D to draw marks with the skin marker 5 at the respective hole positions. This enables drawing of marks indicating the suture positions in forming a valve cusp by suturing the valve cusp material obtained by cutting the pericardium to the aorta. Drawing cannot be performed at positions other than the positions of the hole 11A, the holes 11B, the holes 11C, and the holes 11D, that is, at positions where holes are not open in the template 1, and therefore no matter who draws marks, the marks are drawn at appropriate positions since drawing is performed at predetermined positions when viewed from the lines drawn along the hole 10A and the hole 10B.

Fig. 3 is a diagram illustrating an example of lines and marks drawn on the pericardium by using the template 1 and the skin marker 5. A line 3A is a line drawn by using the hole 10A, and a line 3B is a line drawn by using the hole 10B. A mark 4A is a mark drawn by using the hole 11A, and a mark 4B is a mark drawn by using the hole 11B. A mark 4C is a mark drawn by using the hole 11C, and a mark 4D is a mark drawn by using the hole 11D.

Subsequently, the pericardium is cut along the line 3A and the line 3B to obtain a valve cusp material 6 that is to be formed as a valve cusp. Fig. 4 illustrates an example of the valve cusp material 6 obtained by cutting the pericardium. Since the hole 10A and the hole 10B are not connected to each other, lines that serve as a guide for cutting are not drawn between the end portion of the straight line part 101 of the hole 10A and the end portion of the straight line part 101 of the hole 10B, and between the end portion of the arc part 102 of the hole 10A and the end portion of the arc part 102 of the hole 10B, but these parts are cut so as to connect between them. Until being sutured to the aorta, the valve cusp material 6 obtained from the pericardium is soaked in, for example, physiological saline.

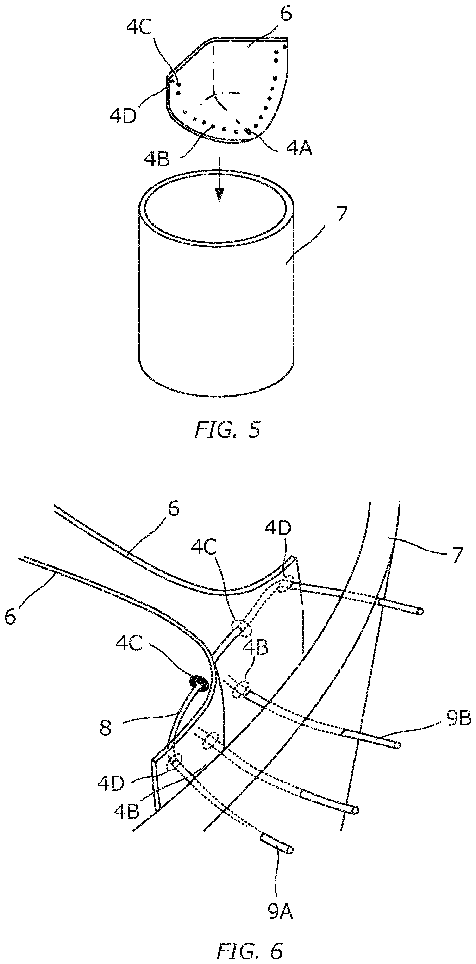

When suturing the valve cusp material 6 to the aorta, the valve cusp material 6 is inserted into the aorta 7 as illustrated in Fig. 5. In the case of assuming a virtual line L1 connecting the mark 4A to the mark 4C illustrated in Fig. 4 in the valve cusp material 6, the outside of the virtual line L1 serves as a suture allowance when suturing the valve cusp material 6 to the aorta 7. A surgeon threads the mark 4A and sutures the valve cusp material 6 to the aorta 7 with the valve cusp material 6 valley-folded along the virtual line L1 when viewed from above, and sequentially sutures the part along the positions of the marks 4B from the mark 4A toward the commissure part. After continuing the suturing to a portion close to the commissure part, the surgeon sticks the needle out of the aorta 7 from a mark 4B that is closest to the mark 4C.

As for the positions of the mark 4C and the mark 4D, the surgeon sticks a needle (not illustrated) on one end side of a thread 8 from the position of the mark 4C to the position of the mark 4C of the adjacent valve cusp material 6, where the thread 8 is different from the thread used for suturing from the suturing start position to the commissure part and has needles at both ends, as illustrated in Fig. 6. The needle on one end side that penetrates the adjacent valve cusp material 6 protrudes out of the aorta 7 from the position of the mark 4D on the adjacent valve cusp material 6, and the needle (not illustrated) on the other end side of the thread 8 protrudes out of the aorta 7 from the position of the mark 4D on the valve cusp material 6.

The surgeon applies a pledget to the outside of the aorta 7 and ties a thread 9A, which has been started from the suturing start position and passed through the mark 4B so as to be out of the aorta 7, to a thread 9B, which passed through the mark 4B of the adjacent valve cusp material 6 so as to be out of the aorta 7, on the pledget and then ties the thread 8, which passed through the marks 4D with both ends out of the blood vessel, on the pledget to fix the valve cusp material 6 to the aorta 7.

According to the template 1 of this embodiment, the hole 11A, the holes 11B, the holes 11C, and the holes 11D cause the mark 4A, the marks 4B, the marks 4C, and the marks 4D to be provided at predetermined ideal positions with the line 3A and the line 3B as a reference. A user of the template 1 only needs to position the pen tip of the skin marker 5 in each hole, and the template 1 is configured so that a mark cannot be drawn at a position other than the position corresponding to each of the above holes in the cut pericardium, by which a mark is prevented from being drawn at an inappropriate position.

### [Modifications]

Although the embodiments of the present invention have been described above, the present invention is not limited to the above-described embodiments and may be carried out in various other modes. For example, the present invention may be carried out by modifying the above embodiments as described below. The embodiments described above and the modifications described below may be respectively combined.

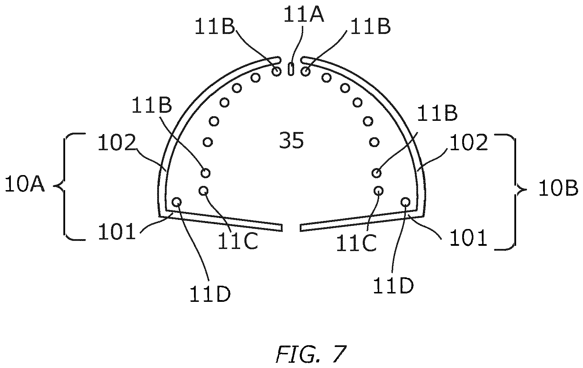

The shapes of the hole 10A and the hole 10B are not limited to the shapes illustrated in Fig. 2, and may be, for example, the shapes illustrated in Fig. 7. The shape of the hole 11C is not limited to the shape illustrated in Fig. 2, and may be different from the shape of the hole 11B, for example. In addition, the shape of the hole 11D may be different from the shape of the hole 10B and the hole 10C. Further, the hole 11C may be different in size from the hole 11B and the hole 11D, and the hole 11D may also be different in size from the hole 11B and the hole 11C.

### Description of Reference Numerals

1: template
2: forming part
3A, 3B: lines
4A, 4B, 4C, 4D: marks
5: skin marker
6: valve cusp material
7: aorta
8: thread
9A, 9B: threads
10A, 10B: holes (first holes)
11A, 11B, 11C, 11D: holes (second holes)
101: straight line part
102: arc part
L1: virtual line

## Claims

1. A template for forming an aortic valve from a pericardium, comprising:
a plurality of first holes (10A, 10B) for drawing lines indicating positions at which a membrane is to be cut, each first hole having a linear straight line part (101) and an arc part (102); and
a plurality of second holes (11A, 11B, 11C, 11D) that are provided at a predetermined distance from the plurality of first holes and are for use in drawing marks at suture positions where the cut membrane is to be sutured,
wherein the plurality of second holes includes:
a hole (11A) for drawing a mark indicating a start position of suturing a valve cusp material formed from the pericardium;
holes (11C) for use in drawing a mark indicating a suture position in suturing the valve cusp material to an adjacent valve cusp;
a plurality of holes (11B), between the hole (11A) for drawing a mark indicating a start position and holes (11C) for use in drawing a mark indicating a suture position in suturing the valve cusp material to an adjacent valve cusp, the positions of the plurality of holes (11B) corresponding to the suture positions in suturing the valve cusp material from the start position of suturing the valve cusp material toward a commissure part;
holes (11D) for use in drawing a mark indicating the suture position in forming the commissure part; wherein the hole (11A) for drawing a mark indicating a start position differs in at least shape or size from the holes for drawing a mark at a position other than the start position.

2. The template according to claim 1, wherein the holes (11C) for use in drawing the mark indicating a suture position in suturing the valve cusp material to an adjacent valve cusp differ in at least shape or size from the plurality of holes (11B) corresponding to the suture positions in suturing the valve cusp material from the start position of suturing the valve cusp material toward a commissure part and from the holes (11D) for use in drawing a mark indicating the suture position in forming the commissure part.

3. The template according to claim 1 or 2, wherein the portion other than the first holes and the second holes is transparent.

## Patentansprüche

1. Schablone zum Bilden einer Aortenklappe aus einem Perikard, umfassend:
eine Vielzahl von ersten Löchern (10A, 10B) zum Zeichnen von Linien, die Positionen angeben, an denen eine Membran geschnitten werden soll, wobei jedes erste Loch einen linearen geradlinigen Teil (101) und einen Bogenteil (102) aufweist; und
eine Vielzahl von zweiten Löchern (11A, 11B, 11C, 11D), die in einem vorbestimmten Abstand von der Vielzahl von ersten Löchern bereitgestellt sind und zur Verwendung beim Zeichnen von Markierungen an Nahtpositionen dienen, an denen die geschnittene Membran genäht werden soll, wobei die Vielzahl von zweiten Löchern Folgendes beinhaltet:
ein Loch (11A) zum Zeichnen einer Markierung, die eine Startposition zum Nähen eines Klappensegelmaterials angibt, aus dem Perikard gebildet ist;
Löcher (11C) zur Verwendung beim Zeichnen einer Markierung, die eine Nahtposition beim Nähen des Klappensegelmaterials an einen benachbarten Ventilsegel angibt;
eine Vielzahl von Löchern (11B) zwischen dem Loch (11A) zum Zeichnen einer Markierung, die eine Startposition angibt, und Löchern (11C) zur Verwendung beim Zeichnen einer Markierung, die eine Nahtposition beim Nähen des Klappensegelmaterials an einen benachbarten Ventilsegel angibt, wobei die Positionen der Vielzahl von Löchern (11B) den Nahtpositionen beim Nähen des Klappensegelmaterials von der Startposition des Nähens des Klappensegelmaterials in Richtung eines Kommissurteils entsprechen;
Löcher (11D) zur Verwendung beim Zeichnen einer Markierung, die die Nahtposition beim Bilden des Kommissurteils angibt; wobei das Loch (11A) zum Zeichnen einer Markierung, die eine Startposition angibt, sich zumindest in Form oder Größe von den Löchern zum Zeichnen einer Markierung an einer anderen Position als der Startposition unterscheidet.

2. Schablone nach Anspruch 1, wobei sich die Löcher (11C) zur Verwendung beim Zeichnen der Markierung, die eine Nahtposition beim Nähen des Klappensegelmaterials an einen benachbarten Klappensegel angibt, zumindest in Form oder Größe von der Vielzahl von Löchern (11B), die den Nahtpositionen beim Nähen des Klappensegelmaterials von der Startposition des Nähens des Klappensegelmaterials in Richtung eines Kommissurteils entsprechen, und von den Löchern (11D) zur Verwendung beim Zeichnen einer Markierung, die die Nahtposition beim Bilden des Kommissurteils angibt, unterscheiden.

3. Schablone nach Anspruch 1 oder 2, wobei der von den ersten Löchern und den zweiten Löchern verschiedene Teil transparent ist.

## Revendications

1. Modèle pour former une valvule aortique à partir d'un péricarde, comprenant :
une pluralité de premiers trous (10A, 10B) pour dessiner des lignes indiquant des positions auxquelles une membrane doit être coupée, chaque premier trou ayant une partie de ligne droite linéaire (101) et une partie d'arc (102) ; et
une pluralité de seconds trous (11A, 11B, 11C, 11D) qui sont prévus à une distance prédéterminée de la pluralité de premiers trous et sont prévus pour être utilisés pour dessiner des marques dans des positions de suture où la membrane coupée doit être suturée,
dans lequel la pluralité de seconds trous comprend :
un trou (11A) pour dessiner une marque indiquant une position de départ pour suturer un matériau de cuspide de valvule formé à partir du péricarde ;
des trous (11C) destinés à être utilisés pour dessiner une marque indiquant une position de suture pour suturer le matériau de cuspide de valvule sur une cuspide de valvule adjacente ;
une pluralité de trous (11B), entre le trou (11A) pour dessiner une marque indiquant une position de départ et des trous (11C) destinés à être utilisés pour dessiner une marque indiquant une position de suture pour suturer le matériau de cuspide de valvule sur une cuspide de valvule adjacente, les positions de la pluralité de trous (11B) correspondant aux positions de suture pour suturer le matériau de cuspide de valvule à partir de la position de départ de suture du matériau de cuspide de valvule vers une partie de commissure ;
des trous (11D) destinés à être utilisés pour dessiner une marque indiquant la position de suture pour former la partie de commissure ; dans lequel le trou (11A) pour dessiner une marque indiquant une position de départ diffère au moins du point de vue de la forme ou de la taille des trous pour dessiner une marque dans une position différente de la position de départ.

2. Modèle selon la revendication 1, dans lequel les trous (11C) destinés à être utilisés pour dessiner la marque indiquant une position de suture pour suturer le matériau de cuspide de valvule sur une cuspide de valvule adjacente diffèrent au moins du point de vue de la forme ou de la taille de la pluralité de trous (11B) correspondant aux positions de suture pour suturer le matériau de cuspide de valvule de la position de départ pour suturer le matériau de cuspide de valvule vers une partie de commissure et des trous (11D) destinés à être utilisés pour dessiner une marque indiquant la position de suture pour former la partie de commissure.

3. Modèle selon la revendication 1 ou 2, dans lequel la partie différente des premiers trous et des seconds trous est transparente.
